# EUROPEAN PATENT APPLICATION

(11) **EP 1 493 437 A1**
(43) Date of publication of application: **05.01.2005**
(21) Application number: 03712784.2
(22) Date of filing: 20.03.2003
(51) Int. Cl.: A61K 31/09, A61K 31/122, A61K 45/00, A61P 3/10, A23L 1/00, A23K 1/00

(54) **COMPOSITIONS FOR DIABETES**

(30) Priority: 20.03.2002 JP 2002077909
(71) Applicant: KANEKA CORPORATION, Osaka-shi, Osaka 530-8288 (JP)
(72) Inventor: FUJII, Kenji, Kobe-shi, Hyogo 651-1202 (JP); KAWABE, Taizo, Himeji-shi, Hyogo 672-8044 (JP); HOSOE, Kazunori, Takasago-shi, Hyogo 676-0025 (JP); HIDAKA, Takayoshi, Kobe-shi, Hyogo 655-0006 (JP)
(74) Representative: VOSSIUS & PARTNER
(86) International application number: PCT/JP2003/003389
(87) International publication number: WO 2003/077895

(57) **Abstract**

The present invention provides a composition for improving glucose tolerance to patients having diabetic hyperglycemia and people having pre-diabetes, i.e., abnormal glucose tolerance. The composition helps control the blood glucose level, prevents diabetic complications, and can be administered over a long term due to its high safety.

The composition containing a coenzyme Q, in particular, a reduced coenzyme Q₁₀, as the main component improves the impaired glucose tolerance of diabetes patients, satisfactorily controls the blood glucose level, and decreases the blood glycosylated hemoglobin level.

## Description

### Technical Field

The present invention relates to a composition for treating diabetes containing coenzyme Q.

### Background Art

Diabetes is one of the most common yet serious lifestyle-related diseases. More than one million patients suffer from diabetes worldwide. Furthermore, the number of people that have pre-diabetes, i.e., a relatively high blood glucose level, far exceeds the number of diabetes patients. Diabetes is a serious disease on a worldwide scale.

There are two types of diabetes known: Type I diabetes caused by insulin hyposecretion (insulin-dependent diabetes) and Type II diabetes caused by a lowered response to insulin (non-insulin-dependent diabetes). Most of the diabetes patients have Type II diabetes, which is frequently caused by an inappropriate lifestyle, such as overeating.

One problem of diabetes is the lack of symptoms indicating hyperglycemia. Thus, the blood glucose level frequently remains uncontrolled over a long term, thereby leading to complications (such as retinopathy, nephropathy, and arteriosclerosis). For example, the most common cause of adult visual impairment in Japan is diabetic retinopathy. The second most common cause of chronic renal failure leading to artificial dialysis in Japan is diabetic nephropathy. Due to the seriousness of these complications, the primary objective of diabetes treatment is to maintain stringent blood glucose control.

The blood glucose of Type I diabetes patients can be highly effectively controlled by insulin injection since insulin hyposecretion is the cause of Type I diabetes. However, insulin injection is not so effective for Type II diabetes patients since Type II diabetes patients have a low response to insulin. Treatments for Type II diabetes patients include diet restriction, in which the intake of excess dietary calories is restricted, combined with kinesitherapy to promote the consumption of calories inside the body. However, diet restriction is difficult to continue in many cases. For those patients who do not show significant progress despite the diet restriction and kinesitherapy, treatment through the administration of drugs for diabetes (sulfonylurea agents, biguanide agents, and the like) is attempted. However, these drugs have the following serious problems. Sulfonylurea agents (SU agents), which have a long history of administration and the second-generation agent of which has been available since the 1970s, are known to cause hypoglycemia resulting from an excessive reduction of the blood glucose level. Moreover, the SU agents produce strong feeling of hunger and thus tend to cause the diet restriction to fail. This often leads to failure in controlling the blood glucose and promotes obesity. Thus, the administration of SU agents having these problems must be tightly controlled by physicians. Biguanide agents (BG agents) have also been used since the 1970s. However, since soon after the release of the biguanide agents, many cases of a side effect, i.e., lactic acidosis, have been reported. Although the administration method and the effectiveness of the BG agents have recently been reassessed, the administration of the BG agent also needs tight control under physicians.

Under these circumstances, the development of new oral agents for diabetes has been attempted, and, for example, α-glucosidase inhibitors and insulin sensitizers have been developed. However, these drugs have a short history, and whether these drugs can be recognized as highly safe and effective agents must await further investigations. In fact, the development of an insulin sensitizer, troglitazone, has been discontinued due to a side effect of hepatopathy.

As is described above, highly safe oral agents for diabetes have been rarely available to diabetes patients. The primary treatment for diabetes is still diet restriction; however, stringent diet restriction is difficult in many actual cases. Although many low-calorie meals for diabetes patients have been developed, they contribute only partially to the treatment of diabetes. Furthermore, since no therapeutic drugs to treat complications characteristic of diabetes are available, prevention of complications is currently done through stringent control of the blood glucose level. Accordingly, treatments of diabetes await highly safe and effective drugs, effective food that can be safely administered during diet restriction, and the like.

Coenzyme Q is an essential ingredient found in a wide diversity of living organisms from bacteria to mammals and is known to function as a constituent of the electron transfer system of mitochondria in the cells of living organisms. Coenzyme Q not only serves as a transfer agent of the electron transfer system by repeating oxidation and reduction in the mitochondria but also has an antioxidative effect. The primary component of the coenzyme Q of human beings is coenzyme Q₁₀, which is a coenzyme Q having a side chain consisting of 10 repeating units.

The important feature of the coenzyme Q₁₀ is high safety. A chronic toxicity test conducted at a daily dose of 1,200 mg/Kg for 52 weeks in rats reported no toxic effects (K. D. Williams et al., J. Agric. Food Chem., 47, 3756-3763, 1999).

With respect to coenzyme Q₁₀, oxidized coenzyme Q₁₀ is widely used as a drug for congestive heart failure in Japan and as a health food in Europe and the United States. Although there is a report that suggests the effectiveness of the oxidized coenzyme Q₁₀ on diabetes (Shimomura, Y. et al., 1981, Rinsho to Kenkyu, 58, 1349-1352), the effectiveness of the oxidized coenzyme Q₁₀ on diabetes patients is not actually known. The reduced coenzyme Q₁₀ is an active form of an oxidized coenzyme Q₁₀ and is readily converted into an oxidized coenzyme Q₁₀ in air by oxidation. Thus, the reduced coenzyme Q₁₀ has not been used in products. We have found before that the oral absorbability of the total coenzyme Q₁₀ can be increased by mixing the reduced coenzyme Q₁₀ with the oxidized coenzyme Q₁₀ compared to when the oxidized coenzyme Q₁₀ is used alone (Japanese Unexamined Patent Application Publication No. 10-109933). However, whether the reduced coenzyme Q₁₀ exhibits a higher effect than the oxidized coenzyme Q₁₀ on diseases such as diabetes has not been clear.

### Summary of the Invention

An object of the present invention is to provide a highly safe oral composition that can satisfactorily control the blood glucose level of diabetes patients.

The present inventors have conducted extensive investigations and found that a composition effective for diabetes can be obtained by using a highly safe coenzyme Q, in particular, a reduced coenzyme Q.

The present invention relates to a composition for treating diabetes, comprising a reduced coenzyme Q represented by formula (1) as an effective component: 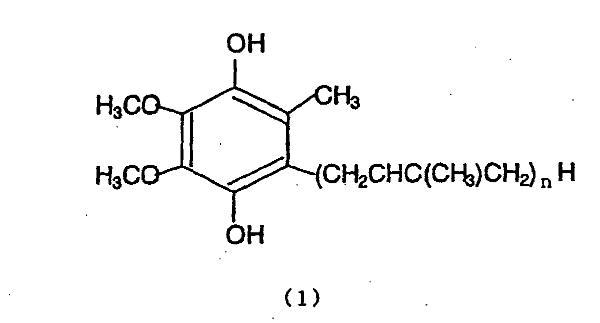 (wherein n represents an integer between 1 and 12).

The present invention also relates to a composition for treating diabetes, comprising the reduced coenzyme Q described above and an oxidized coenzyme Q represented by formula (2) as effective components: 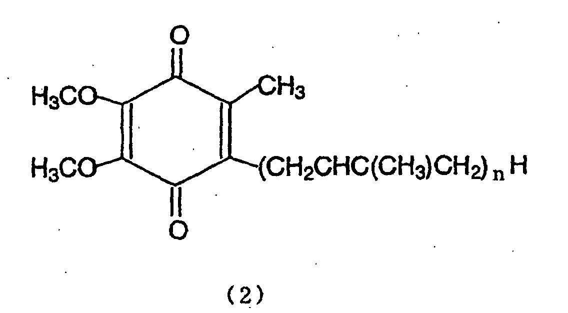 (wherein n represents an integer between 1 and 12).

The present invention also relates to the above-described compositions in which the coenzyme Q is a coenzyme Q₁₀; to the above-described compositions further containing a therapeutic drug for diabetes; and to the above-described compositions in which the therapeutic drug for diabetes is a sulfonylurea agent, a sulfonamide agent, a biguanide agent, an α-glucosidase inhibitor, an insulin sensitizer, or insulin.

The present invention also relates to a drug, a functional food, and animal feedstuff containing the above-described composition for treating diabetes.

The present invention also relates to a method for controlling the blood glucose level and a method for preventing diabetic complications using the above-described compositions for treating diabetes.

### Detailed Disclosure of the Invention

The present invention will now be described in detail.

A coenzyme Q is represented by either formula (1): 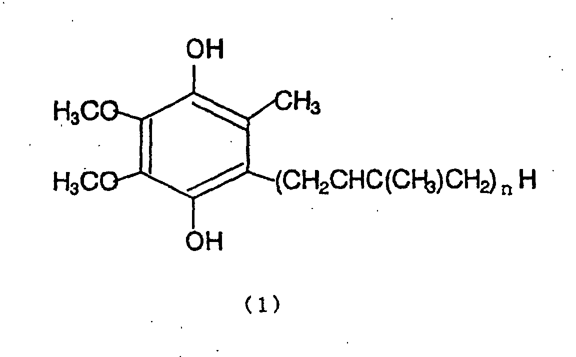 (wherein n represents an integer between 1 and 12), or formula (2): 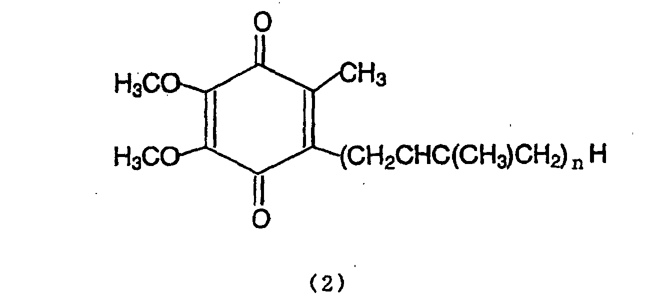 (wherein n represents an integer between 1 and 12). The coenzyme Q represented by formula (1) is a reduced coenzyme Q and the coenzyme Q represented by formula (2) is an oxidized coenzyme Q.

The process for obtaining the oxidized coenzyme Q and the reduced coenzyme Q is not particularly limited. An example of such a process includes the steps of preparing a coenzyme Q by a conventional method, such as synthesis, fermentation, or extraction from natural materials, and condensing an oxidized coenzyme Q fraction or a reduced coenzyme Q fraction of the eluate obtained by chromatography. The oxidized coenzyme Q can be prepared by a conventional process. The reduced coenzyme Q may be prepared by adding a common reductant, such as sodium borohydride or sodium dithionite (sodium hydrosulfite), to the coenzyme Q obtained as above, reducing the coenzyme Q by a conventional method to prepare a reduced coenzyme Q, and condensing the resulting reduced coenzyme Q by chromatography. The reduced coenzyme Q can be prepared by interaction of the above-described reductant with a commonly available high-purity coenzyme Q.

As shown in formulae (1) and (2), the coenzyme Q used in the present invention has a side chain having one to twelve repeating units ("n" in the formulae). A coenzyme Q having a side chain having ten repeating units, i.e., a coenzyme Q₁₀, is particularly preferred.

The composition for treating diabetes according to the present invention contains either the above-described reduced coenzyme Q as an effective component or the above-described reduced coenzyme Q and the above-described oxidized coenzyme Q as effective components.

The process for obtaining the composition for treating diabetes is not particularly limited. For example, either the reduced coenzyme Q prepared as above or the reduced coenzyme Q and the oxidized coenzyme Q prepared as above may be dissolved in an appropriate common solvent, such as isopropyl alcohol, acetone, or ether, so as to obtain a composition containing a predetermined amount of the reduced coenzyme Q. Alternatively, the reduced coenzyme Q in solid form may be used. Alternatively, the reduced coenzyme Q in solid form may simply be mixed with the oxidized coenzyme Q in solid form. Alternatively, a mixture of the oxidized coenzyme Q and the reduced coenzyme Q obtained in the process for producing the coenzyme Q described above can be directly used. The composition of the present invention can be directly prepared by controlling the duration of the reduction of the above-described commonly available high-purity coenzyme Q, the type of the reductant, or the amount of the reductant.

In view of effectiveness, costs, and the like, the composition for treating diabetes according to the present invention preferably contains 0.001 to 20 percent by weight, and more preferably 0.01 to 10 percent by weight of the coenzyme Q to the total amount of the composition.

The reduced coenzyme Q content is preferably at least 60 percent by weight, and more preferably at least 80 percent by weight of the total amount of the coenzyme Q to achieve higher efficacy.

The composition for treating diabetes according to the present invention may contain a drug for treating diabetes in addition. Examples of the drug for treating diabetes include sulfonylurea agents, sulfonamide agents, biguanide agents, α-glucosidase inhibitors, insulin sensitizers, and insulin.

The content of the drug for treating diabetes is not particularly limited and may be appropriately determined according to the usage, such as drugs, functional foods, and animal feedstuffs.

The composition for treating diabetes according to the present invention improves glucose tolerance and facilitates the control of blood glucose levels, for example. Thus, the composition for treating diabetes is effective for preventing the onset of diabetes, treating diabetes, and preventing diabetic complications.

The composition for treating diabetes of the present invention can also be used in methods for controlling the blood glucose level and methods for preventing diabetic complications.

The above composition for treating diabetes of the present invention can be used in, for example, drugs for diabetes, functional foods and food materials for diabetes, and feedstuffs for diabetic animals. Here, the term "functional foods" refers to products that are not drugs but can be taken orally to maintain or improve health. Examples of the functional foods include oral supplements, foods for specified health use, health foods, and dietary supplements.

The composition for treating diabetes can be used in any applications that involve oral administration of the coenzyme Q or oral administration of the coenzyme Q and the drug.

When the composition for treating diabetes of the present invention is used as drugs, functional foods, animal feedstuffs, or the like, the dosage form is not particularly limited. For example, the composition for treating diabetes may be used as a powdered agent, a granular agent prepared by mixing a binder, a coated powder agent prepared by coating powder particles with a coating agent, or a capsule containing the powdered agent, the granular agent, or the coated powder agent. The composition for treating diabetes may be blended with natural oil, oily higher fatty acids, higher fatty acid monoglycerides, a surfactant, or a mixture of these to prepare a soft capsule containing the resulting oily mixture. Here, a material mainly composed of gelatin or other water-soluble polymer may also be used. Moreover, the term "capsule" includes microcapsules.

In particular, for functional food applications, the coenzyme Q may be incorporated into conventional foods for diabetes.

The composition for treating diabetes preferably contains an antioxidant for preventing oxidation of the reduced coenzyme Q. However, the antioxidant may not be necessary depending on the form of dosage. Examples of the antioxidant include citric acid and derivatives thereof, vitamin C and derivatives thereof, lycopene, vitamin A, carotenoids, vitamin B and derivatives thereof, flavonoids, polyphenols, glutathione, selenium, sodium thiosulfate, vitamin E and derivatives thereof, superoxide dismutase (SOD), glutathione peroxidase, glutathione-S-transferase, glutathione reductase, catalase, ascorbate peroxidase, and combinations of these.

A chelating agent may be used to also prevent oxidation. Examples of the chelating agent include ethylenediaminetetraacetic acid and salts thereof, ethylenediaminediacetic acid and salts thereof, hydroxyiminodiacetic acid and salts thereof, hydroxyethylethylenediaminetetraacetic acid and salts thereof, diethylenetriaminepentaacetic acid and salts thereof, nitrilotriacetic acid and salts thereof, triethylenetetraaminehexaacetic acid and salts thereof, dicarboxymethyl glutamic acid tetrasodium salt, dihydroxymethylglycine, 1,3-propanediaminetetraacetic acid and salts thereof, 1,3-diamino-2-hydroxypropanetetraacetic acid and salts thereof, gluconic acid sodium salt, hydroxyethylidenediphosphonic acid, nitrilotris, phosphonobutanetricarboxylic acid, and mixtures thereof.

Furthermore, the above-described antioxidants may be used in combination with the chelating agents.

The composition for treating diabetes of the present invention may contain adequate amounts of pharmaceutically acceptable and food-hygiene-acceptable materials in addition to the above-described reduced coenzyme Q according to conventional processes. Examples of the pharmaceutically acceptable and food-hygiene-acceptable materials include, but are not limited to, excipients, disintegrants, lubricants, binders, coating agents, coloring agents, coagulation inhibitors, absorption promoters, solubilizing agents, stabilizing agents, health food materials, dietary supplement materials, and vitamins.

Examples of the excipient include, but are not limited to, sucrose, lactose, glucose, cornstarch, mannitol, crystalline cellulose, calcium phosphate, and calcium sulfate.

Examples of the disintegrants include, but are not limited to, starch, agar, calcium citrate, calcium carbonate, sodium hydrogencarbonate, dextrin, crystalline cellulose, carboxymethylcellulose, and tragacanth.

Examples of the lubricants include, but are not limited to, talc, magnesium stearate, polyethylene glycol, silica, and hydrogenated vegetable oil.

Examples of the binders include, but are not limited to, ethyl cellulose, methyl cellulose, hydroxypropylmethyl cellulose, tragacanth, shellac, gelatin, gum arabic, polyvinyl pyrrolidone, polyvinyl alcohol, polyacrylic acid, polymethacrylic acid, and sorbitol.

Examples of the coating agents include, but are not limited to, gum arabic, opadry, prunella, castor wax, carboxyvinyl polymer, carmellose, hydrous silicon dioxide, magnesium silicate, vinyl acetate resin, stearic acid, cetanol, and hydroxypropylmethyl cellulose.

Examples of the coloring agents include, but are not limited to, those which are permitted to be added to drugs and foods.

Examples of the coagulation inhibitors include, but are not limited to, stearic acid, talc, light silicic anhydride, and hydrous silicon dioxide.

Examples of the absorption promoters include, but are not limited to, surfactants such as higher alcohols, higher fatty acids, and glycerin fatty acid esters.

Examples of the solubilizing agents include, but are not limited to, organic acids such as fumaric acid, succinic acid, and malic acid.

Examples of the stabilizing agents include, but are not limited to, benzoic acid, sodium benzoate, and ethyl parahydroxybenzoate.

Examples of the health food materials include, but are not limited to, Chinese herb medicines (e.g., irei-to, unkei-to, unsei-in, ogi-kenchu-to, oren-gedoku-to, oren-to, ka-kkon-to, kami-kihi-to, kami-syoyo-san, kam-baku-taiso-to, kikyou-to, kihi-to, kumi-binro-to, keigai-rengyo-to, keihika-syakuyakudaiou-to, keihi-ka-syakuyaku-to, keihi-karyukotsu-borei-to, keishi-to, keishi-ninjin-to, keishi-bukuryo-gan, keihi-to, koso-san, goko-to, goshaku-san, gosha-jinki-gan, gorin-san, saikan-to, saiko-ka-ryukotsuborei-to, saiko-keihi-kankyo-to, saiko-keishi-to, saiko-seikan-to, saiboku-to, sairei-to, sansonin-to, jiin-koka-to, shigyaku-san, shikunshi-to, shimotsu-to, sha-kanzo-to, shakuyaku-kanzo-to, juzen-daiho-to, jumi-haidoku-to, sho-kenchu-to, sho-saiko-to, sho-seiryu-to, shofu-san, shin'iseihai-to, shimpi-to, shimbu-to, seijo-bufu-to, seisho-ekkito, seishin-renshi-in, seihai-to, sokei-kakketsu-to, daio-kanzo-to, daio-botampi-to, dai-kenchu-to, dai-saiko-to, dai-saiko-to-kyo-daiou, dai-jouki-to, dai-bofu-to, ji-daboku-ippo, choi-joki-to, choto-san, choyo-to, chorei-to, chorei-to-go-shimotsu-to, tsu-do-san, tokaku-joki-to, toki-inshi, toki-kenchu-to, toki-shakuyaku-san, toki-to, nichin-to, nyoshin-san, ninjin-to, ninjin-yoei-to, haiao-san-kyu-to, bakumondo-to, hachimi-jio-gan, hange-koboku-to, hange-shashin-to, byakko-ka-ninjin-to, bukuryo-in, bukuryo-in-go-hange-koboku-to, heii-san, boi-ogi-to, bofu-tsusho-san, hochu-ekki-to, mao-to, mao-bushi-saishin-to, makyo-kan-seki-to, mashinin-gan, moku-boi-to, yoku-kan-san, yokukan-san-kachimpi-hange, rikkushi-to, rikko-san, ryutan-shakan-to, ryokan-kyo-mi-shin-ge-nin-to, and rokumi-gan); teas (e.g., green tea, brown rice tea, powdered green tea, natural leaf tea, roasted tea, roasted tea, jasmine tea, oolong tea, red tea, black tea, floral tea, blue tea, and white tea); herbs (e.g., Italian parsley, elecampane, olive, oregano, cardoon, chamomiles, curry plant, catnip, caraway, christmas rose, crimson clover, cornflower, common mallow, salad burnet, santolina, cinnamon, jasmine, stevia, sage, linden, scented geranium, St. John's wort, soapwort, Solomon's seal, thyme, tansy, chervil, chive, nasturtium, jujube, basil, honeysuckle, hyssop, flax, fennel, foxglove, black hollyhock, French marigold, betony, heliotrope, bergamot, hemp agrimony, rue, pot marigold, borage, white horehound, myrtle, mullein, marjoram, mint, yarrow, lavender, lady's bedstraw, lemongrass, lemon verbena, lemon balm, rose, rosemary, rocket, wild strawberry, wild pansy, and forgetmenot); propolis; gingko leaves; green juice; and extracts thereof.

Examples of the dietary supplement materials include, but are not limited to, amino acids, metal ions, proteins, saccharides, fatty acids, yeast extracts, vegetable extracts, fish extracts, fruits, and fruit extracts.

Examples of the vitamins include, but are not limited to, vitamins A, B, C, D, and E.

The content, dosage form, preservation method, and preservation form of the reduced coenzyme Q during the production of the composition containing the reduced coenzyme Q for treating diabetes according to the present invention may be adequately selected according to applications, such as drugs, health food, food, animal drugs, or animal feedstuff.

### Brief Description of the Drawings

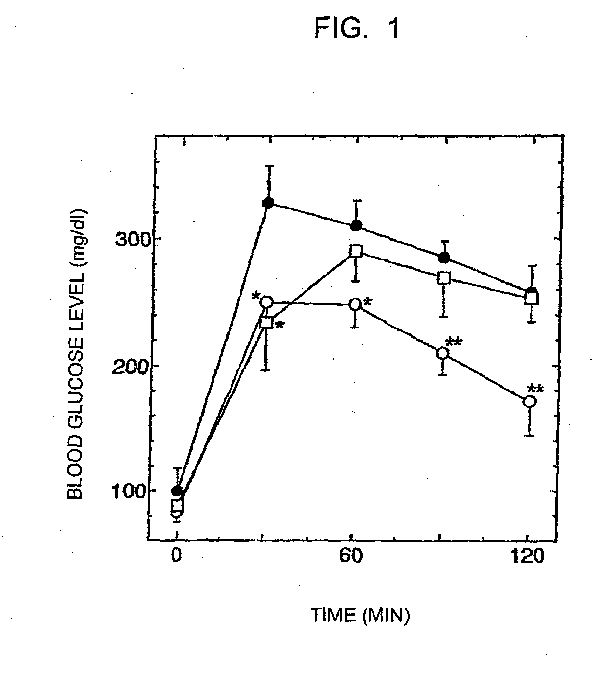

Fig. 1 is a graph showing the glucose tolerance of GK rats fed with a feedstuff containing coenzyme Q₁₀ for four weeks. The ordinate indicates the blood glucose level (mg/dl), and the abscissa indicates the time (minutes) elapsed since the glucose load. The average values ±standard deviation in four rats are plotted. Solid circles indicate a control group; open circles indicate a group fed with the reduced coenzyme Q₁₀ (containing 2% of the oxidized coenzyme Q₁₀); and open squares indicate a group fed with the oxidized coenzyme Q₁₀. Single asterisk and double asterisk represent significant differences relative to the control group at significance levels of 5% and less than 1%, respectively (Dunnet's test).

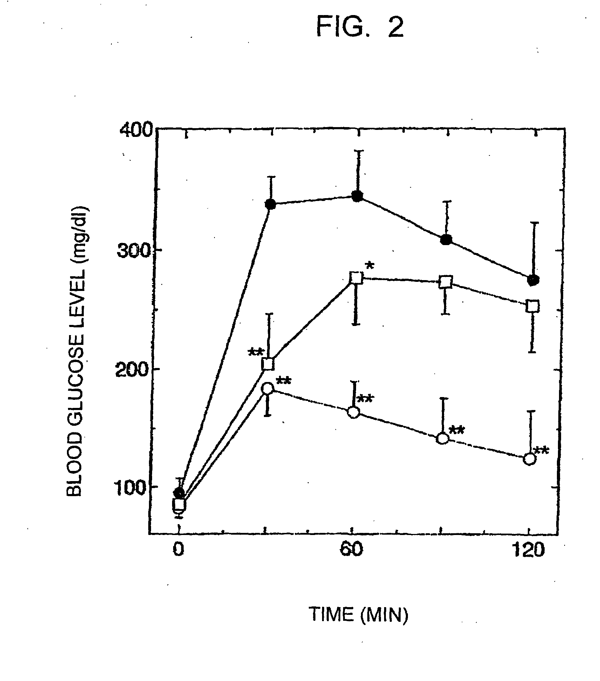

Fig. 2 is a graph showing the glucose tolerance of GK rats fed with a feedstuff containing coenzyme Q₁₀ for six weeks. The ordinate indicates the blood glucose level (mg/dl), and the abscissa indicates the time (minutes) elapsed since the glucose load. The average values ±standard deviation of four rats are plotted. Solid circles indicate a control group; open circles indicate a group fed with the reduced coenzyme Q₁₀ (containing 2% of the oxidized coenzyme Q₁₀); and open squares indicate a group fed with the oxidized coenzyme Q₁₀. Single asterisk and double asterisk represent significant differences relative to the control group at significance levels of 5% and less than 1%, respectively (Dunnet's test).

### Best Mode for Carrying Out the Invention

The present invention will now be described in detail by way of nonlimiting examples.

### (EXAMPLE 1) Effects on Model Rats Having Type II Diabetes

The improvement effects of the reduced coenzyme Q₁₀ and the oxidized coenzyme Q₁₀ on the glucose tolerance were examined using model GK rats (5 weeks old, male) having Type II diabetes. The rats were fed ad libitum with a powdered feedstuff containing 0.1 percent by weight of the reduced coenzyme Q₁₀ (containing 2 percent by weight of the oxidized coenzyme Q₁₀) or a feedstuff containing 0.1 percent by weight of the oxidized coenzyme Q₁₀. In order to avoid the reduced coenzyme Q₁₀ in the feedstuff mixture from decreasing due to oxidation, the feedstuff was completely replaced with a new one every two days. The control group was fed with a powdered feedstuff free of coenzyme Q₁₀. The glucose tolerance test was conducted four weeks and six weeks after the feeding of the feedstuff mixture was started (n = 4 in each group). In the glucose tolerance test, a 50% glucose solution was orally administered at a dose of 4 ml/kg, and the blood glucose level was measured before the administration and 30 minutes, 60 minutes, 90 minutes, and 120 minutes after the administration. The blood glucose level in blood collected from the tail vein was determined with a simple blood glucose meter. The results are shown in Figs. 1 and 2.

Fig. 1 shows the results of the glucose tolerance test after four weeks of administration. The increase in blood glucose level in the group fed with the oxidized coenzyme Q₁₀ 30 minutes after the glucose load was significantly inhibited in comparison with the control group. This fact shows that the glucose tolerance was improved. The blood glucose level in the group fed with the reduced coenzyme Q₁₀ sharply decreased in comparison with the oxidized coenzyme Q₁₀ group at all measurement times beginning with 30 minutes after the glucose load. This fact shows that the glucose tolerance was further improved.

Fig. 2 shows the results after six weeks of administration. The glucose tolerance in the group fed with the oxidized coenzyme Q₁₀ showed further improvements in comparison with that in after four weeks. The increase in the blood glucose level after the glucose load was inhibited in comparison with the oxidized coenzyme Q₁₀. The increase in blood glucose level in the group fed with the reduced coenzyme Q₁₀ was strongly inhibited at all measurement times. The blood glucose level was 200 mg/dl or less at all measurement points. This fact shows that the glucose tolerance was improved to a greater extent in comparison with that after four weeks of administration.

These results show that the glucose tolerance of GK rats can be improved by the administration of the oxidized coenzyme Q₁₀. Furthermore, the test results also show that the glucose tolerance of GK rats can be further improved by the administration of the reduced coenzyme Q₁₀ in comparison with the administration of the oxidized form. Interestingly, the improvement patterns of the glucose tolerance in the group fed with the oxidized coenzyme Q₁₀ after four weeks and six weeks of administration were the same. In particular, the increase in blood glucose level 30 minutes after the glucose load was inhibited; however, the blood glucose level kept increasing until about an hour after the glucose load and subsequently became almost steady, during which no large difference was observed in comparison with the control group. In contrast, the glucose tolerance improvement patterns in the group fed with the reduced coenzyme Q₁₀ after four weeks and six weeks of administration peaked 30 minutes after the load and decreased with time. The blood glucose level in the group fed with the reduced coenzyme Q₁₀ 30 minutes after the peak was significantly lower than that of the control group. This fact shows that the glucose tolerance was clearly improved. It was a surprise to find that the pattern of improvements on the glucose tolerance achieved by the reduced coenzyme Q₁₀ was different from that achieved by the oxidized coenzyme Q₁₀. In the previous investigations, we discovered that the oral absorbability of the reduced coenzyme Q₁₀ is different from that of the oxidized coenzyme Q₁₀ (Japanese Unexamined Patent Application Publication No. 10-109933). This time we discovered that the improvement pattern of the glucose tolerance in GK rats was different between the oxidized form and the reduced form. The difference in absorbability alone cannot account for such differences. Thus, it can be presumed that the reduced coenzyme Q₁₀ has different effects from those of the oxidized coenzyme Q₁₀ *in vivo*.

### (EXAMPLE 2) Effects on Blood Glycosylated Hemoglobin

The blood glycosylated hemoglobin (hemoglobin A1c) level of GK rats fed for 12 weeks was measured as in EXAMPLE 1. The results are shown in Table 1. The hemoglobin A1c level in the group fed with the reduced coenzyme Q₁₀ showed a significant decrease, i.e., about 78% of the blood hemoglobin A1c level of the control group. In contrast, although the hemoglobin A1c level in the group fed with the oxidized coenzyme Q₁₀ showed a decrease, i.e., 92% of that of the control group, the decrease was not significant and was smaller than the decrease achieved by the group fed with the reduced coenzyme Q₁₀. Glycosylated hemoglobin levels are clinically important parameters for controlling blood glucose levels. The fact that the group fed with the reduced coenzyme Q₁₀ showed a significantly low level-indicates that the blood glucose level of GK rats was satisfactorily controlled by the administration of this substance. The group fed with the oxidized coenzyme Q₁₀ only showed a slight decrease, which was not as significant as that achieved by the group fed with the reduced coenzyme Q₁₀. This result concerning the glycosylated hemoglobin level is presumably attributable to the insufficient improvements in glucose tolerance shown in EXAMPLE 1.

**Table 1**

| Blood hemoglobin A1c level in GK rats | |
|---|---|
| Group | Hemoglobin A1c level (%) |
| Control group | 5.33±0.48 |
| | (100) |
| Group fed with reduced coenzyme Q₁₀ | 4.17±0.15 |
| | (78**) |
| Group fed with oxidized coenzyme Q₁₀ | 4.93±0.42 |
| | (92) |

| | |
|---|---|
| **Significantly different from control group at p < 0.01 (Student's t-test). | |

### Industrial Applicability

The present invention can provide a useful composition for maintaining satisfactory blood glucose level control in diabetes patients and persons having pre-diabetes, i.e., abnormal glucose tolerance. The composition for treating diabetes of the present invention containing coenzyme Q as the main component, which is highly safe, can be administered over a long term and is highly useful. Furthermore, the present invention can provide a composition useful for treating diabetes of not only human beings but also pet animals.

## Claims

1. A composition for treating diabetes, comprising a reduced coenzyme Q represented by formula (1) as an effective component: 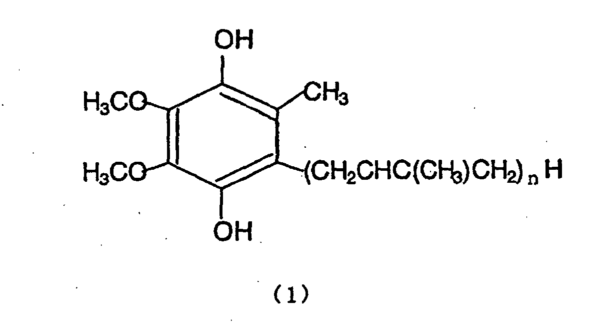 (wherein n represents an integer between 1 and 12).

2. A composition for treating diabetes, comprising a reduced coenzyme Q and an oxidized coenzyme Q represented by formula (2) as effective components: 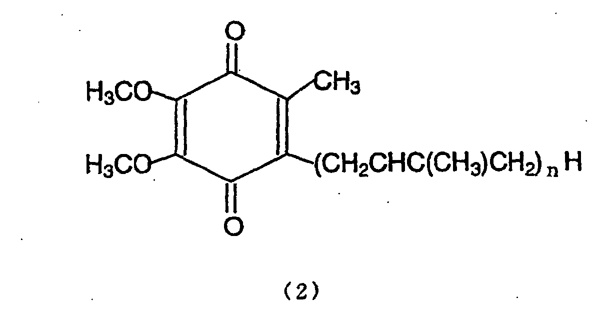 (wherein n represents an integer between 1 and 12).

3. The composition for treating diabetes according to claim 1 or 2, wherein the coenzyme Q is a coenzyme Q₁₀.

4. The composition for treating diabetes according to one of claims 1 to 3, further comprising a therapeutic drug for diabetes.

5. The composition for treating diabetes according to claim 4, wherein the therapeutic drug for diabetes is a sulfonylurea agent, a sulfonamide agent, a biguanide agent, an α-glucosidase inhibitor, an insulin sensitizer, or insulin.

6. A drug for treating diabetes, comprising the composition for treating diabetes according one of claims 1 to 5.

7. A functional food for treating diabetes, comprising the composition for treating diabetes according one of claims 1 to 5.

8. An animal feedstuff for treating diabetes, comprising the composition for treating diabetes according one of claims 1 to 5.

9. A method for controlling a blood glucose level, comprising using the composition for treating diabetes according one of claims 1 to 5.

10. A method for preventing a diabetic complication, comprising using the composition for treating diabetes according one of claims 1 to 5.
